# FASCICULE DE BREVET EUROPEEN

(11) **EP 3 549 192 B1**
(45) Date de publication et mention de la délivrance du brevet: **09.06.2021**
(21) Numéro de dépôt: 17817788.7
(22) Date de dépôt: 30.11.2017
(51) Int. Cl.: H01M 10/052, H01M 10/0568, H01M 10/0569

(54) **AMELIORATION DE LA CONDUCTIVITE IONIQUE D'ELECTROLYTE A BASE DE SELS DE LITHIUM D'IMIDAZOLATE**
VERBESSERUNG DER IONENLEITFÄHIGKEIT EINES ELEKTROLYTEN AUF BASIS VON LITHIUM-IMIDAZOLAT-SALZEN
IMPROVING THE ION CONDUCTIVITY OF AN ELECTROLYTE BASED ON LITHIUM IMIDAZOLATE SALTS

(30) Priorité: 02.12.2016 FR 1661855
(43) Date de publication de la demande: 09.10.2019
(73) Titulaire: Arkema France, 92700 Colombes (FR); Université de Tours, 37000 Tours (FR)
(72) Inventeur: SCHMIDT, Grégory, 69700 Saint Andeol le Château (FR); ANOUTI, Mérièm, 37550 St Avertin (FR); LEMORDANT, Daniel, 38530 Pontcharra/Breda (FR); TIMPERMAN, Laure, 37320 Esvres (FR); BERHAUT, Christopher, 86470 Lavausseau (FR)
(74) Mandataire: Arkema Patent
(86) Numéro de dépôt international: PCT/FR2017/053297
(87) Numéro de publication internationale: WO 2018/100297

(56) Documents cités:
- WO-A1-2010/023413
- WO-A1-2015/136201
- US-A1- 2011 229 769

## Description

### DOMAINE DE L'INVENTION

La présente invention concerne une composition électrolytique comprenant au moins un électrolyte à base de sel d'imidazolate de lithium, et son utilisation dans les batteries Li-ion.

La présente invention concerne également l'utilisation d'un mélange de solvants spécifiques pour améliorer la conductivité ionique d'électrolyte à base de sel d'imidazolate de lithium.

### ARRIERE-PLAN TECHNIQUE

Une batterie lithium-ion comprend au moins une électrode négative (anode), une électrode positive (cathode), un séparateur et un électrolyte, l'électrolyte étant constitué généralement d'un sel de lithium dissous dans un solvant qui est typiquement un mélange de carbonates organiques, afin d'avoir un bon compromis entre la viscosité et la constante diélectrique. Des additifs peuvent être ajoutés pour améliorer la stabilité des sels d'électrolyte.

Parmi les sels les plus utilisés figure l'hexafluorophosphate de lithium (LiPF₆), qui possède plusieurs des nombreuses qualités requises pour un usage en batterie mais présente le désavantage de se dégrader sous forme de gaz d'acide fluorhydrique par réaction avec l'eau et sous l'effet de la température. Cela pose des problèmes de sécurité, notamment dans le contexte de l'utilisation de batteries lithium-ion pour les véhicules particuliers.

Récemment, d'autres sels ont été développés, tels que le LiTDI (le 1-trifluorométhyl-4,5-dicarbonitrile-imidazolate de lithium) et le LiPDI (1-pentafluoroéthyl-4,5-dicarbonitrile-imidazolate de lithium). Ces sels présentent l'avantage de posséder seulement 3 atomes de fluor liés fortement au carbone, donc moins labiles, en lieu et place des 6 liaisons phosphore-fluor fragiles de LiPF₆. Ces sels ont également l'avantage de ne pas réagir avec l'eau pour générer de l'acide hydrofluorhydrique.

Par ailleurs, le document WO 2010/023413 montre que ces sels présentent des conductivités de l'ordre de 6 mS/cm, une très bonne dissociation entre l'anion imidazolate et le cation lithium. Mais cette conductivité ionique mesurée dans les solvants dits classiques des électrolytes, que sont les mélanges de carbonates, est trop faible pour une utilisation dans les batteries dites de type puissance (par exemple pour les tablettes).

Il existe donc un besoin pour de nouvelles compositions d'électrolytes, notamment à base de lithium d'imidazolate, ayant une conductivité ionique élevée.

### DESCRIPTION DE L'INVENTION

L'invention concerne une composition électrolytique comprenant (de préférence constituée de):
- au moins un sel de lithium de formule (A) ; 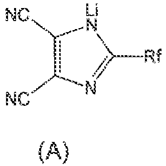 dans laquelle Rf représente un atome de fluor, un groupement nitrile, un groupement alkyle éventuellement fluoré ou perfluoré ayant de 1 à 5 carbones, un groupement alcoxy éventuellement fluoré ou perfluoré ayant de 1 à 5 carbones, ou un groupement oxa-alcoxy éventuellement fluoré ou perfluoré ayant 1 à 5 carbones ; et
- le mélange de solvant suivant: carbonate d'éthylène, γ-butyrolactone, et propanoate de méthyle (CH₃CH₂COOCH₃).

La demanderesse a découvert que l'utilisation du mélange ternaire de solvant : carbonate d'éthylène/γ-butyrolactone/propanoate de méthyle permet avantageusement d'améliorer la conductivité ionique des électrolytes à base de sel de lithium de formule (A), notamment à base de LiTDI. Les compositions selon l'invention peuvent avantageusement être utilisées comme électrolytes dans les batteries dites de type puissance.

En outre, la faible viscosité et la large gamme de température à laquelle les mélanges ternaires selon l'invention sont à l'état liquide offrent avantageusement une gamme de température de fonctionnement plus étendue de la batterie. Le mélange ternaire selon l'invention permet avantageusement de pouvoir utiliser les sels de formule (A), et notamment le 2-trifluonométhyl-4,5-dicyano-imidazolate de lithium (LiTDI), dans des applications à haute et à basse température.

Selon l'invention, la composition électrolytique susmentionnée peut comprendre un sel de formule (A), ou un mélange de sels de formule (A).

A titre d'exemples, on peut notamment citer les groupes Rf suivants : F, CF₃, CHF₂, CH₂F, C2HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃, CF₂OCF₃, C₅F₁₁OCH₃, CF₂OC₂H₅, CF₂OC₂H₄OCH₃, CF₂OC₂H₄OC₂H₅, CF₂OCH₂OCF₂, CF(CF₃)OCH₃, CF(CF₃)OC₂H₅, CF(CF₂)OC₂H₄OCH₃ ou CF(CF₃)OC₂H₂F₃.

Rf est choisi de préférence dans le groupe constitué de : F, CF₃, CHF₂, CH₂F, C2HF₄, C₂H₂F₃, C₂H₃F₂, C₂F₅, C₃F₇, C₃H₂F₅, C₃H₄F₃, C₄F₉, C₄H₂F₇, C₄H₄F₅ et C₅F₁₁,

Rf représente de préférence un groupement CF₃.

Selon un mode de réalisation, la proportion massique du carbonate d'éthylène est supérieure à 8%, de préférence supérieure à 20%, avantageusement supérieure à 25%, et en particulier supérieure à 30%, et préférentiellement supérieure à 45% de la masse totale du mélange ternaire carbonate d'éthylène/γ-butyroiactone/propanoate de méthyle dans la composition électrolytique.

Selon un mode de réalisation, la proportion massique du γ-butyrolactone est supérieure à 8%, de préférence supérieure à 20%, avantageusement supérieure à 25%, et en particulier supérieure à 30%, et préférentiellement supérieure à 45% de la masse totale du mélange ternaire carbonate d'éthylène/γ-butyrolactonelpropanoate de méthyle dans la composition électrolytique.

Selon un mode de réalisation, la proportion massique du propanoate de méthyle est supérieure à 8%, de préférence supérieure à 20%, avantageusement supérieure à 25%, et en particulier supérieure à 30%, et préférentiellement supérieure à 45% de la masse totale du mélange ternaire carbonate d'éthyiène/γ-butyrolactonelpropanoate de méthyle dans la composition électrolytique.

Selon l'invention, le ratio massique carbonate d'éthylène/γ-butyrolactone/ propanoate de méthyle dans la composition électrolytique peut être compris entre 1/1/1 et 1/1/10 (de préférence entre 1/1/1 et 1/1/2), ou entre 1/1/1 et 1/10/1 (de préférence entre 1/1/1 et 1/2/1), ou entre 1/1/1 et 10/1/1 (de préférence entre 1/1/1 et 2/1/1).

Selon un mode de réalisation, le ratio massique carbonate d'éthylène/γ-butyrolactonelpropanoate de méthyle dans la composition électrolytique est 1/1/1.

Selon un mode de réalisation, le ratio massique carbonate d'éthylène/γ-butyrolactonelpropanoate de méthyle dans la composition électrolytique est 1/1/2.

Selon un mode de réalisation, le ratio massique carbonate d'éthylène/γ-butyrolactone/propanoate de méthyle dans la composition électrolytique est 1/2/1.

Selon un mode de réalisation, le ratio massique carbonate d'éthylène/γ-butyrolactonelpropanoate de méthyle dans la composition électrolytique est 2/1/1.

Selon un mode de réalisation, la composition électrolytique comprend (de préférence est constituée de):
- au moins un sel de lithium de formule (A) : 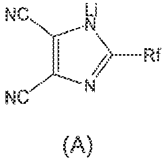 dans laquelle Rf est choisi dans le groupe constitué de F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃, CF₂OCF₃, C₅F₁₁OCH₃, CF₂OC₂H₅, CF₂OC₂H₄OCH₃, CF₂OC₂H₄OC₂H₅, CF₂OCH₂OCF₂, CF(CF₃)OCH₃, CF(CF₃)OC₂H₅, CF(CF₃)OC₂H₄OCH₃ et CF(CF₃)OC₂H₂F₃, de préférence Rf est CF₃; et
- le mélange de solvant suivant: carbonate d'éthylène, γ-butyrolactone, et propanoate de méthyle, de préférence le ratio (en masse) carbonate d'éthylène/γ-butyrolactone/propanoate de méthyle dans la composition électrolytique étant compris entre 1/1/1 et 1/1/10 (de préférence entre 1/1/1 et 1/1/2), ou entre 1/1/1 et 1/10/1 (de préférence entre 1/1/1 et 1/2/1), ou entre 1/1/1 et 10/1/1 (de préférence entre 1/1/1 et 2/1/1).

Selon un mode de réalisation, la concentration de sel de lithium de formule (A), en particulier dans laquelle Rf représente CF₃, dans la composition électrolytique susmentionnée est comprise entre 0,01 et 10 mol/L, de préférence entre 0,05 et 2 mol/L, préférentiellement entre 0,1 et 1,5 mol/L, en particulier entre 0,5 et 1,2 mol/L, avantageusement entre 0,5 et 1 mol/L. De préférence, la concentration de sel de lithium de formule (A), en particulier dans laquelle Rf représente CF₃, dans la composition électrolytique est de 1 mol/L.

La quantité de sel de lithium de formule (A) dissous dans le mélange de solvants décrits ci-dessus peut varier entre 0,01 et 10 mol/l, de préférence entre 0,05 et 2 mol/L, préférentiellement entre 0,1 et 1,5 mol/L, et en particulier entre 0,5 et 1 mol/L.

De préférence, le(s) sel(s) de lithium de formule (A) représente(nt) entre 2% et 100% en poids de la totalité des sels présents dans la composition électrolytique, de préférence entre 25% et 100% en poids, et préférentiellement entre 50% et 100% en poids.

De préférence, la composition électrolytique ne comprend pas d'autres sels de lithium.

Selon un mode de réalisation, la proportion massique de sel(s) de lithium de formule (A) dans la composition électrolytique est comprise entre 0,1% et 50%, de préférence entre 0,5% et 20% en masse par rapport à la masse totale de la composition.

Selon un mode de réalisation, la proportion massique du mélange de solvant dans la composition électrolytique est comprise entre 20% et 99,9%, de préférence entre 50% et 99,5% en masse par rapport à la masse totale de la composition.

La composition électrolytique selon l'invention peut comprendre au moins un additif, par exemple choisi dans le groupe constitué du carbonate de fluoroéthylène (FEC), du carbonate de vinylène, du 4-vinyl-1,3-dioxolan-2-one, de la pyridazine, de la vinyl pyridazine, de la quinoline, de la vinyl quinoline, du butadiène, du sébaconitrile, du LiB(C₂O₄)₂, du nitrate de lithium, des alkyldisulfure, du fluorotoluène, du 1,4-diméthoxytétrafluorotoluène, du t-butylphenol, du di-t-butylphenol, du tris(pentafluorophenyl)borane, des oximes, des époxydes aliphatiques, des biphényls halogénés, des acides métacryliques, du carbonate d'allyle éthyle, de l'acétate de vinyle, de l'adipate de divinyle, de l'acrylonitrile, du 2-vinylpyridine, de l'anhydride maléïque du cinnamate de méthyle, des phosphonates, des composés silane contenant un vinyle, du 2-cyanofurane, et de leurs mélanges, l'additif électrolytique étant de préférence le carbonate de fluoroéthylène (FEC).

Selon un mode de réalisation, la composition électrolytique selon l'invention peut comprendre des additifs, par exemple le carbonate de fluoroéthylène ou le 1,3-Dioxol-2-one (carbonate de vinylène).

La teneur en additifs dans la composition électrolytique selon l'invention peut être comprise entre 0,1% et 5%, de préférence entre 1 % et 5% en masse par rapport à la masse totale de la composition.

Selon un mode de réalisation, la composition électrolytique selon l'invention a une viscosité cinématique supérieure à 1,5 mPa.s, de préférence supérieure à 1,7 mPa.s, en particulier compris entre 1,7 mPa.s et 6 mPa.s, à une température de 25°C.

La viscosité de la composition électrolytique peut être mesurée à 25°C à l'aide d'un densimètre de préférence Anton Parr (par exemple le modèle 60/602, Anton Parr, France) couplé à un viscosimètre à bille (par exemple le Lovis 2000/ME, Anton Parr, France) permettant la mesure de la viscosité cinématique. La température de chaque appareil peut être contrôlée avec une incertitude de ± 0,02 °C. Le densimètre peut par exemple être préalablement étalonné à l'aide d'eau plate prévue à cet effet et d'air sec à pression atmosphérique, tandis que de l'eau distillée peut être utilisée pour étalonner le viscosimètre.

Selon un mode de réalisation, la composition électrolytique de l'invention a une conductivité ionique supérieure ou égale à 7 mS.cm⁻¹, en particulier comprise entre 7 et 20 mS.cm⁻¹, à une température supérieure ou égale à 25°C, et de préférence entre 25°C et 100°C, en particulier entre 25°C et 80°C, la composition électrolytique comprenant de préférence un sel de formule (A) à une concentration de 1 mol/L.

En particulier, la composition électrolytique selon l'invention a une conductivité ionique supérieure à 7,5 mS.cm⁻¹, de préférence supérieure à 8 mS.cm⁻¹, et préférentiellement supérieure ou égale à 8.5 mS.cm⁻¹ à 25°C.

La conductivité ionique peut être mesurée par tout moyen connu. Elle peut notamment être mesurée à l'aide d'un conductimètre Crison (GLP 31) multifréquences (1000-5000 Hz), ou d'un conductimètre BioLogic multivoies composé d'un module de mesure d'impédances (MCM 10) connecté à un module de Peltier (WTSH 10) permettant notamment des mesures de conductivité entre -40°C et 150°C. Les températures de mesures peuvent être fixées par un bain thermostaté JULABO avec une précision de 0,2°C. Avant chaque mesure la cellule de conductivité peut être étalonnée en utilisant des étalons de KCl par exemple à trois concentrations différentes. Les cellules de mesure constituées d'électrodes parallèles en platine peuvent être scellées en boite à gants protégeant les échantillons d'une exposition à l'air et à toutes traces d'humidité.

La présente invention concerne également l'utilisation de ladite composition électrolytique susmentionnée comme électrolyte de batteries Li-ion, en particulier de batteries Li-ion d'appareils nomades, par exemple les téléphones portables ou les ordinateurs portables, de véhicules électriques, de stockage d'énergie renouvelable par exemple le photovoltaïque ou l'éolien.

De préférence, la présente invention concerne l'utilisation de ladite composition électrolytique susmentionnée comme électrolyte de batteries Li-ion (en particulier de batteries Li-ion d'appareils nomades, par exemple les téléphones portables ou les ordinateurs portables, de véhicules électriques, de stockage d'énergie renouvelable par exemple le photovoltaïque ou l'éolien) dans une gamme de température comprise entre -60°C et 130°C.

La présente demande concerne également une cellule électrochimique comportant une électrode négative, une électrode positive, et la composition électrolytique telle que décrite ci-dessus, notamment interposée entre l'électrode négative et l'électrode positive. La cellule électrochimique peut aussi comprendre un séparateur, dans lequel est imprégnée la composition d'électrolyte telle que définie ci-dessus.

La présente invention concerne également une batterie comprenant au moins une cellule électrochimique telle que décrite ci-dessus. Lorsque la batterie comprend plusieurs cellules électrochimiques selon l'invention, lesdites cellules peuvent être assemblées en série et/ou en parallèle.

Dans le cadre de l'invention, par électrode négative, on entend l'électrode qui fait office d'anode, quand la batterie débite du courant (c'est-à-dire lorsqu'elle est en processus de décharge) et qui fait office de cathode lorsque la batterie est en processus de charge.

L'électrode négative comprend typiquement un matériau électrochimiquement actif, éventuellement un matériau conducteur électronique, et éventuellement un liant.

Dans le cadre de l'invention, on entend par « matériau électrochimiquement actif », un matériau capable d'insérer de manière réversible des ions.

Dans le cadre de l'invention, on entend par « matériau conducteur électronique » un matériau capable conduire les électrons.

Selon un mode de réalisation, l'électrode négative de la cellule électrochimique comprend, comme matériau électrochimiquement actif, du graphite, du lithium, un alliage de lithium, un titanate de lithium de type Li₄Ti₅O₁₂ ou TiO₂, du silicium ou un alliage de lithium et silicium, un oxyde d'étain, un composé intermétallique de lithium, ou un de leurs mélanges.

L'électrode négative peut comprendre du lithium, celui-ci peut alors être constitué d'un film de lithium métallique ou d'un alliage comprenant du lithium. Un exemple d'électrode négative peut comprendre un film de lithium vif préparé par laminage, entre des rouleaux, d'un feuillard de lithium.

Dans le cadre de l'invention, par électrode positive, on entend l'électrode qui fait office de cathode, quand la batterie débite du courant (c'est-à-dire lorsqu'elle est en processus de décharge) et qui fait office d'anode lorsque la batterie est en processus de charge.

L'électrode positive comprend typiquement un matériau électrochimiquement actif, éventuellement un matériau conducteur électronique, et éventuellement un liant.

Dans un autre mode de réalisation, l'électrode positive de la cellule électrochimique comprend un matériau électrochimiquement actif choisi parmi le dioxyde de manganèse (MnO₂), l'oxyde de fer, l'oxyde de cuivre, l'oxyde de nickel, les oxydes composites lithium-manganèse (par exemple LiₓMn₂O₄ ou LiₓMnO₂), les oxydes compositions lithium-nickel (par exemple LiₓNiO₂), les oxydes compositions lithium-cobalt (par exemple LiₓCoO₂), les oxydes composites lithium-nickel-cobalt (par exemple LiNi_{1-y}Co_{y}O₂), les oxydes composites lithium-nickel-cobalt-mangenèse (par exemple LINiₓMn_{y}Co_{z}O₂ avec x+y+z = 1), les oxydes composites lithium-nickel-cobalt-manganèse enrichis en lithium (par exemple Li₁₊ₓ(NiMnCo)₁₋ₓO₂), les oxydes composites de lithium et de métal de transition, les oxydes composites de lithium-manganèse-nickel de structure spinelle (par exemple LiₓMn_{2-y}Ni_{y}O₄), les oxydes de lithium-phosphore de structure olivine (par exemple LiₓFePO₄, LiₓFe_{1-y}Mn_{y}PO₄ ou LiₓCoPO₄), le sulfate de fer, les oxydes de vanadium, et leurs mélanges.

De préférence, l'électrode positive est comprend un matériau électrochimiquement actif choisi parmi LiCoO₂, LiFePO₄ (LFP), LiMnₓCo_{y}N_{z}O₂ (NMC, avec x+y+z = 1), LiFePO₄F, LiFeSO₄F, LiNiCoAlO₂ et leurs mélanges.

Le matériau de l'électrode positive peut aussi comprendre, outre le matériau électrochimiquement actif, un matériau conducteur électronique comme une source de carbone, incluant, par exemple, du noir de carbone, du carbone Ketjen®, du carbone Shawinigan, du graphite, du graphène, des nanotubes de carbone, des fibres de carbone (tels les fibres de carbone formées en phase gazeuse (VGCF), du carbone non-poudreux obtenu par carbonisation d'un précurseur organique, ou une combinaison de deux ou plus de ceux-ci. D'autres additifs peuvent aussi être présents dans le matériau de l'électrode positive, comme des sels de lithium ou des particules inorganiques de type céramique ou verre, ou encore d'autres matériaux actifs compatibles (par exemple, du soufre).

Le matériau de l'électrode positive peut aussi comprendre un liant. Des exemples non-limitatifs de liants comprennent les liants polymères polyéthers linéaires, ramifiés et/ou réticulé (par exemple, des polymères basés sur le poly(oxyde d'éthylène) (PEO), ou le poly(oxyde de propylène) (PPO) ou d'un mélange des deux (ou un copolymère EO/PO), et comprenant éventuellement des unités réticulables), des liants solubles dans l'eau (tels que SBR (caoutchouc styrène-butadiène), NBR (caoutchouc acrylonitrile-butadiène), HNBR (NBR hydrogéné). CHR (caoutchouc d'épichlorohydrine), ACM (caoutchouc d'acrylate)), ou des liants de type polymères fluorés (tels que PVDF (fluorure de polyvinylidène), PTFE (polytétrafluoroéthylène), et leurs combinaisons. Certains liants, comme ceux solubles dans l'eau, peuvent aussi comprendre un additif comme le CMC (carboxyméthylcellulose).

La présente invention a également pour objet l'utilisation d'un mélange de solvant: carbonate d'éthylène, γ-butyrolactone, et propanoate de méthyle, pour améliorer la conductivité ionique d'électrolyte à base de sels d'imidazolate de lithium, de préférence de sel de formule (A) susmentionnée, et encore plus préférentiellement du sel LiTDI (2-trifluorométhyl-4,5-dicyano-imidazolate de lithium).

Selon un mode de réalisation, l'invention concerne l'utilisation d'un mélange de solvants : carbonate d'éthylène, γ-butyrolactone, et propanoate de méthyle, pour améliorer la conductivité ionique d'électrolyte à base de sels d'imidazolate de lithium, de préférence de sel de formule (A) susmentionnée, dans une batterie Li-ion.

Dans le cadre de l'invention, par « comprise entre x et y » ou « allant de x à y », on entend un intervalle dans lequel les bornes x et y sont incluses. Par exemple, la gamme «comprise entre 25 et 100% » inclus notamment les valeurs 25 et 100%.

Tous les modes de réalisation décrits ci-dessus peuvent être combinés les uns avec les autres.

Les exemples suivants illustrent l'invention sans toutefois la limiter.

### EXEMPLES

### Exemple 1 : Mesure des conductivités ioniques

Les mesures de conductivité ont été réalisées à l'aide de deux instruments différents. Le premier est un conductimètre Crison (GLP 31) multifréquences (1000-5000 Hz). Les températures de mesures ont été fixées par un bain thermostaté JULABO avec une précision de 0,2°C. Avant chaque mesure la cellule de conductivité a été étalonnée en utilisant des étalons de KCl à trois concentrations différentes. Le deuxième instrument ayant été utilisé est un conductimètre BioLogic multivoies composé d'un module de mesure d'impédances (MCM 10) connecté à un module de Peltier (WTSH 10) permettant des mesures de conductivité entre -40°C et 150°C. Les cellules de mesure constituées d'électrodes parallèles en platine ont été scellées en boite à gants protégeant les échantillons d'une exposition à l'air et à toutes traces d'humidité.

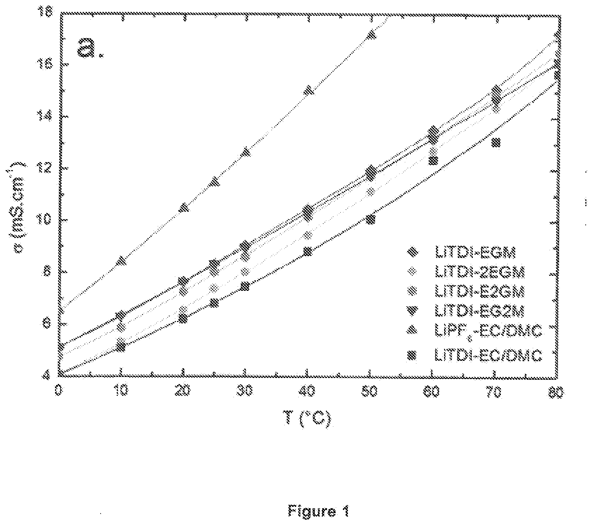

La figure 1 montre la conductivité ionique en fonction de la température de mélanges xEyGzM (avec x, y et z représentant les proportions massiques de chaque solvant, E = carbonate d'éthylène, G = γ-butyrolactone, et M = propanoate de méthyle), en présence de LiTDI à 1 mol/L.

Les compositions électrolytiques selon l'invention présentent avantageusement de meilleures conductivités ioniques que le LiDTI dans un mélange classique carbonate d'éthylène (EC) et carbonate de diméthyle (DMC).

En particulier, une nette amélioration de la conductivité ionique est observée lors du passage d'EC/DMC (rapport massique 1/1) à EGM (x=y=z=1, rapport massique 1/1/1) comme mélange de solvants pour LiTDI. En effet, d'une valeur de 6,81 mS.cm⁻¹ dans EC/DMC (rapport massique 1/1), la conductivité d'une solution de LiTDI à 1 mol.L⁻¹ à 25 °C monte à 8,32 mS.cm⁻¹ dans EGM (x=y=z=1, rapport massique 1/1/1). Sur la gamme de température étudiée ici, l'électrolyte LiTDI-EGM (x=y=z=1, rapport massique 1/1/1) permet notamment l'obtention des meilleures conductivités suivi de près par LiTDI-E2GM (x=z=1 et y =2, rapport massique 1/2/1) et LiTDI-EG2M (x=y=1 et z=2, rapport massique 1/1/2).

Grâce à sa permittivité relative élevée (58 ± 5) et à sa faible viscosité en présence de LiTDI à 1 mol.L⁻¹ (2,4 mPa.s à 25 °C), le mélange EGM (x=y=z=1, rapport massique 1/1/1) permet l'obtention de conductivités plus hautes pour LiTDI (8,5 mS.cm⁻¹ à 25 °C) que les mélanges ayant déjà été utilisés en présence de ce sel de lithium tels que EC/DMC (rapport massique 1/1) (6,8 mS.cm⁻¹), 3EC/7DEC (3,39 mS.cm⁻¹) (rapport volumique 3/7) ou 8EC/16DMC/1DME (rapport massique 8/16/1) (6,13 mS.cm⁻¹) (DEC et DME signifiant respectivement le carbonate de diéthyle et le diméthoxyéthane).

### Exemple 2 : viscosité

La viscosité cinématique des compositions a été mesurée à 25°C à l'aide d'un densimètre de préférence Anton Parr modèle 60/602 (Anton Parr, France) couplé à un viscosimètre à bille Lovis 2000/ME (Anton Parr, France). Le densimètre a été préalablement étalonné à l'aide d'eau plate prévue à cet effet et d'air sec à pression atmosphérique, tandis que de l'eau distillée a été utilisée pour étalonner le viscosimètre.

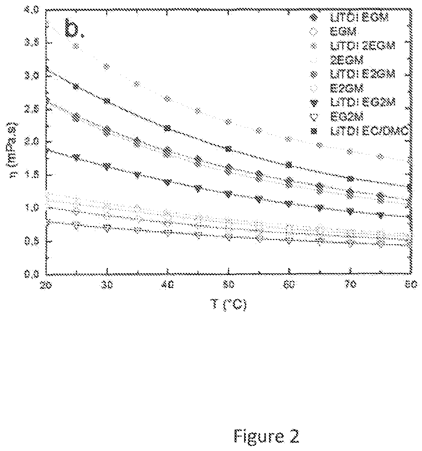

La Figure 2 décrit la viscosité cinématique en fonction de la température, pour les compositions suivantes : LiTDI-EGM, LiTDI-2EGM, EGM, 2EGM, LiTDI-E2GM, E2GM, LiTDI-EG2M, EG2M, LiTDI-EC/DMC.

### Exemple 3 : calorimétrie différentielle à balayage

Les résultats de calorimétrie différentielle à balayage ou DSC (*Differential Scanning Calorimetry*) ont été obtenus à l'aide du DSC 4000 PerkinElmer. Les échantillons, initialement à 30°C, ont dans un premier temps été refroidis jusqu'à -40°C puis chauffés jusqu'à 70°C avant d'être de nouveau refroidis à -60°C, puis finalement chauffés jusqu'à atteindre 150°C. Chaque balayage a été réalisé à 2°C.min⁻¹ et suivi d'une isotherme de 1 min.

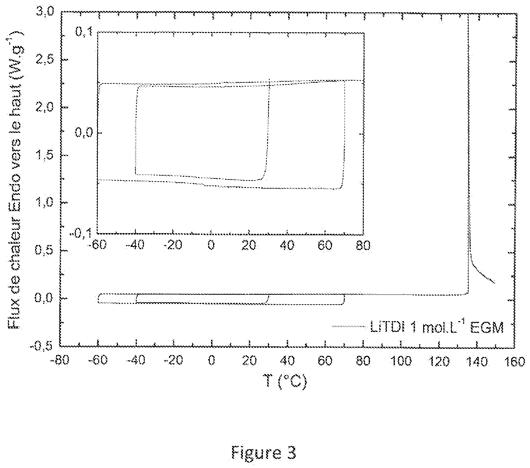

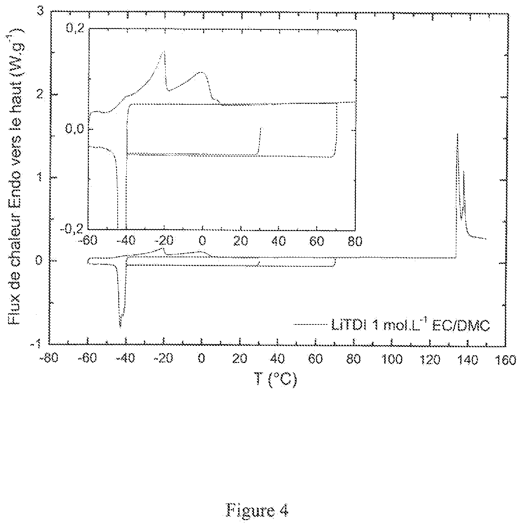

Les analyses DSC entre -60°C et 150°C des électrolytes LiTDI-EGM (1/1/1 en masse) (selon l'invention) et LiTDI-EC/DMC (1/1) (exemple comparatif) sont présentées dans les figures 3 et 4.

La figure 3 est un thermogramme DSC de l'électrolyte LiTDI-EGM (avec LiTDI à une concentration de 1 mol/L), obtenu avec une vitesse de balayage de 2 °C.min⁻¹. La figure comprend un zoom sur la gamme de température entre -60°C et 80°C.

La figure 4 est un thermogramme DSC de l'électrolyte LiTDI-EC/DMC (avec LiTDI à une concentration de 1 mol/L) obtenu avec une vitesse de balayage de 2 °C.min⁻¹. La figure comprend un zoom sur la gamme de température entre -60°C et 80°C.

Les deux électrolytes peuvent supporter des températures allant jusqu'à 130°C environ. Au-delà les électrolytes entrent en ébullition causant la rupture de la capsule DSC. La composition LiTDI-EGM (rapport massique 1/1/1) (selon l'invention) apparait comme légèrement plus stable à haute température. Malgré sa volatilité, le MP, en plus de la présence de GBL, permet une bonne tenue de l'électrolyte LiTDI-EGM (rapport massique 1/1/1) à très faible température. En effet, aucun changement de phase n'est observé pour ce dernier alors que LiTDI-EC/DMC (comparatif) connait une solidification dès -40 °C. Aucun pic de solidification ou de fusion n'est observé dans le cas de LiTDI-EGM (rapport massique 1/1/1) suggérant une stabilité à de plus basses températures.

## Revendications

1. Composition électrolytique comprenant :
- au moins un sel de lithium de formule (A) : 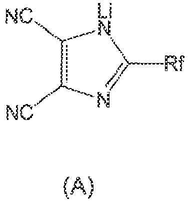 dans laquelle Rf représente un atome de fluor, un groupement nitrile, un groupement alkyle éventuellement fluoré ou perfluoré ayant de 1 à 5 carbones, un groupement alcoxy éventuellement fluoré ou perfluoré ayant de 1 à 5 carbones ou un groupement oxa-alcoxy éventuellement fluoré ou perfluoré ayant 1 à 5 carbones ; et
- le mélange de solvant suivant: carbonate d'éthylène, γ-butyrolactone, et propanoate de méthyle.

2. Composition selon la revendication 1, dans laquelle Rf représente F, CF₂, CHF₂, CH₂F, C2HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃, CF₂OCF₃, C₅F₁₁OCH₃, CF₂OC₂H₅, CF₂OC₂H₄OCH₃, CF₂OC₂H4OC₂H₅, CF₂OCH₂OCF₃, CF(CF₃)OCH₃, CF(CF₃)OC₂H₅, CF(CF₃)OC₂H₄OCH₃ ou CF(CF₃)OC₂H₂F₃.

3. Composition selon la revendication 2, dans laquelle Rf représente CF₃.

4. Composition selon l'une quelconque des revendications 1 à 3, dans laquelle la concentration de sel de lithium de formule (A) dans la composition est comprise entre 0,01 et 10 mol/L, de préférence entre 0,05 et 2 mol/L, préférentiellement entre 0,1 et 1,5 mol/L, en particulier entre 0,5 et 1,2 mol/L, avantageusement entre 0,5 et 1 mol/L.

5. Composition selon l'une quelconque des revendications 1 à 4, dans laquelle le ratio massique carbonate d'éthylène/y-bulyrolactone/propanoate de méthyle est compris entre 1/1/1 et 1/1/10, ou entre 1/1/1 et 1/10/1, ou entre 1/1/1 et 10/1/1.

6. Composition selon la revendication 5, dans laquelle, le ratio massique carbonate d'éthylènely-butyrolactonelpropanoate de méthyle est 1/1/1, 1/1/2, 1/2/1 ou 2/1/1.

7. Composition selon l'une quelconque des revendications 1 à 6, ayant une conductivité ionique supérieure ou égale à 7 mS.cm⁻¹, en particulier comprise entre 7 et 20 mS.cm⁻¹, à une température supérieure ou égale à 25°C.

8. Composition selon l'une quelconque des revendications 1 à 7, ayant une viscosité cinématique supérieure à 1,5 mPa.s, de préférence supérieure à 1,7 mPa.s, en particulier compris entre 1,7 mPa.s et 6 mPa.s, à une température de 25°C.

9. Utilisation d'une composition selon l'une quelconque des revendications 1 à 8, dans une batterie Li-ion.

10. Cellule électrochimique comportant une électrode négative, une électrode positive, et une composition d'électrolyte selon l'une quelconque des revendications 1 à 8, de préférence interposée entre l'électrode négative et l'électrode positive.

11. Batterie comprenant au moins une cellule électrochimique selon la revendication 10.

12. Utilisation d'un mélange ternaire de solvant : carbonate d'éthylène, γ-butyrolactone, et propanoate de méthyle, pour améliorer la conductivité ionique d'électrolyte à base de sels d'imidazolate de lithium, de préférence de sel de formule (A), et encore plus préférentiellement de 2-trffluorométhyl-4,5-dicyano-imidazolate de lithium (LiTDI).

13. Utilisation selon la revendication 12, dans une batterie Li-ion.

## Patentansprüche

1. Elektrolytzusammensetzung, umfassend:
- mindestens ein Lithiumsalz der Formel (A): 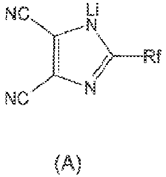 in der Rf für ein Fluoratom, eine Nitrilgruppe, eine gegebenenfalls fluorierte oder perfluorierte Alkylgruppe mit 1 bis 5 Kohlenstoffatomen, eine gegebenenfalls fluorierte oder perfluorierte Alkoxygruppe mit 1 bis 5 Kohlenstoffatomen oder eine gegebenenfalls fluorierte oder perfluorierte Oxaalkoxygruppe mit 1 bis 5 Kohlenstoffatomen steht; und
- die folgende Lösungsmittelmischung: Ethylencarbonat, γ-Butyrolacton und Propansäuremethylester.

2. Zusammensetzung nach Anspruch 1, wobei Rf für F, CF₃, CHF₂, CH₂F, C2HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃, CF₂OCF₃, C₅F₁₁OCH₃, CF₂OC₂H₅, CF₂OC₂H₄OCH₃, CF₂OC₂H₄OC₂H₅, CF₂OCH₂OCF₃, CF (CF₃) OCH₃, CF (CF₃) OC₂H₅, CF (CF₃) OC₂H₄OCH₃ oder CF (CF₃) OC₂H₂F₃ steht.

3. Zusammensetzung nach Anspruch 2, wobei Rf für CF₃ steht.

4. Zusammensetzung nach einem der Ansprüche 1 bis 3, wobei die Konzentration des Lithiumsalzes der Formel (A) in der Zusammensetzung zwischen 0,01 und 10 mol/l, vorzugsweise zwischen 0,05 und 2 mol/l, bevorzugt zwischen 0,1 und 1,5 mol/l, insbesondere zwischen 0,5 und 1,2 mol/l, vorteilhafterweise zwischen 0,5 und 1 mol/l, liegt.

5. Zusammensetzung nach einem der Ansprüche 1 bis 4, wobei das Ethylencarbonat/γ-Butyrolacton/Propansäuremethylester-Massenverhältnis zwischen 1/1/1 und 1/1/10 oder zwischen 1/1/1 und 1/10/1 oder zwischen 1/1/1 und 10/1/1 liegt.

6. Zusammensetzung nach Anspruch 5, wobei das Ethylencarbonat/γ-Butyrolacton/Propansäuremethylester-Massenverhältnis 1/1/1, 1/1/2, 1/2/1 oder 2/1/1 beträgt.

7. Zusammensetzung nach einem der Ansprüche 1 bis 6 mit einer Ionenleitfähigkeit größer oder gleich 7 mS.cm⁻¹, insbesondere zwischen 7 und 20 mS.cm⁻¹, bei einer Temperatur größer oder gleich 25 °C.

8. Zusammensetzung nach einem der Ansprüche 1 bis 7 mit einer kinematischen Viskosität von mehr als 1,5 mPa.s, bevorzugt mehr als 1,7 mPa.s, insbesondere zwischen 1,7 mPa.s und 6 mPa.s, bei einer Temperatur von 25 °C.

9. Verwendung einer Zusammensetzung nach einem der Ansprüche 1 bis 8 in einer Li-Ionen-Batterie.

10. Elektrochemische Zelle mit einer negativen Elektrode, einer positiven Elektrode und einer Elektrolytzusammensetzung nach einem der Ansprüche 1 bis 8, welche vorzugsweise zwischen der negativen Elektrode und der positiven Elektrode angeordnet ist.

11. Batterie mit mindestens einer elektrochemischen Zelle nach Anspruch 10.

12. Verwendung einer ternären Lösungsmittelmischung:
Ethylencarbonat, γ-Butyrolacton und Propansäuremethylester zur Verbesserung der Ionenleitfähigkeit eines Elektrolyts auf Basis von Lithiumimidazolatsalzen, vorzugsweise Salz der Formel (A) und noch weiter bevorzugt Lithium-2-trifluormethyl-4,5-dicyanoimidazolat (LiTDI).

13. Verwendung nach Anspruch 12 in einer Li-Ionen-Batterie.

## Claims

1. Electrolytic composition comprising:
- at least one lithium salt of formula (A): 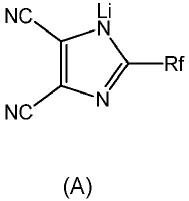 wherein Rf represents a fluorine atom, a nitrile group, an optionally fluorinated or perfluorinated alkyl group having from 1 to 5 carbons, an optionally fluorinated or perfluorinated alkoxy group having from 1 to 5 carbons or an optionally fluorinated or perfluorinated oxa-alkoxy group having from 1 to 5 carbons; and
- the following solvent mixture: ethylene carbonate, y- butyrolactone, and methyl propanoate.

2. Composition according to claim 1, in which Rf represents F, CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₃F₅OCF₃, C₂F₄OCF₃, C₂H₂F₂OCF₃, CF₂OCF₃, C₅F₁₁OCH₃, CF₂OC₂H₅, CF₂OC₂H₄OCH₃, CF₂OC₂H₄OC₂H₅, CF₂OCH₂OCF₃, CF(CF₃)OCH₃, CF(CF₃)OC₂H₅, CF(CF₃)OC₂H₄OCH₃ or CF(CF₃)OC₂H₂F₃.

3. Composition according to claim 2, in which Rf represents CF₃.

4. Composition according to any of claims 1 to 3, in which the concentration of lithium salt of formula (A) in the composition is comprised between 0.01 and 10 mol/L, preferably between 0.05 and 2 mol/L, preferentially between 0.1 and 1.5 mol/L, in particular between 0.5 and 1.2 mol/L, advantageously between 0.5 and 1 mol/L.

5. Composition according to any of claims 1 to 4, in which the mass ratio of ethylene carbonate/y-butyrolactone/methyl propanoate is comprised between 1/1/1 and 1/1/10, or between 1/1/1 and 1/10/1, or between 1/1/1 and 10/1/1.

6. Composition according to claim 5, in which the mass ratio of ethylene carbonate/y-butyrolactone/methyl propanoate is 1/1/1, 1/1/2, 1/2/1 or 2/1/1.

7. Composition according to any of claims 1 to 6, having an ionic conductivity greater than or equal to 7 mS/cm⁻¹, in particular comprised between 7 and 20 mS/cm⁻¹, at a temperature greater than or equal to 25°C.

8. Composition according to any of claims 1 to 7, having a kinematic viscosity of greater than 1.5 mPa.s, preferably greater than 1.7 mPa.s, in particular comprised between 1.7 mPa.s and 6 mPa.s, at a temperature of 25°C.

9. Use of a composition according to any of claims 1 to 8 in a Li-ion battery.

10. Electrochemical cell comprising a negative electron, a positive electron, and an electrolytic composition according to any one of claims 1 to 8, preferably interposed between the negative electrode and the positive electrode.

11. Battery comprising at least one electrochemical cell according to claim 10.

12. Use of a ternary solvent mixture: ethylene carbonate, γ-butyrolactone, and methyl propanoate, to improve the ionic conductivity of an electrolyte based on lithium imidazolate salts, preferably salt of formula (A), and yet more preferably of LiTDI (lithium 2-trifluoromethyl-4,5-dicyano-imidazolate) salt.

13. Use according to claim 12, in a Li-ion battery.
